# EUROPEAN PATENT APPLICATION

(11) **EP 4 238 552 A1**
(43) Date of publication of application: **06.09.2023**
(21) Application number: 21886862.8
(22) Date of filing: 28.10.2021
(51) Int. Cl.: A61K 9/00, A61K 47/12, A61K 47/44, A61K 47/40, A61K 31/4709, A61K 31/196, A61P 31/04, A61P 29/00

(54) **OPHTHALMIC COMPOSITION**

(30) Priority: 29.10.2020 KR 20200142134
(71) Applicant: Taejoon Pharmaceutical Co., Ltd., Seoul 04401 (KR)
(72) Inventor: LEE, Joon Youb, Seoul 04401 (KR); KANG, Hae Kyeong, Yongin-si Gyeonggi-do 17008 (KR); HONG, Hee Kyung, Suwon-si Gyeonggi-do 16505 (KR)
(74) Representative: HGF
(86) International application number: PCT/KR2021/015342
(87) International publication number: WO 2022/092868

(57) **Abstract**

The present invention relates to an ophthalmic composition with excellent stability.

The ophthalmic composition according to the present invention may be useful as eye drops which show excellent appearance stability, secure long-term storage stability, and are easy to store while exhibiting excellent antibacterial and anti-inflammatory effects.

## Description

### Technical Field

The present invention relates to an ophthalmic composition containing moxifloxacin and bromfenac with excellent stability.

### Background Art

Bacterial eye diseases or inflammatory eye diseases are ophthalmic diseases which occur frequently enough so that a treatment market thereof accounts for about 10% of a global eye disease treatment market. Its types include bacterial conjunctivitis, tarsadenitis, keratitis, blepharitis, and scleritis.

Meanwhile, moxifloxacin, which is a representative antibiotic with an antibacterial effect, and bromfenac, which is a representative non-steroidal anti-inflammatory drug with an anti-inflammatory effect, have been prepared as eye drops and used as a therapeutic agent for the bacterial eye diseases or the inflammatory eye diseases.

However, when both moxifloxacin and bromfenac are prepared in a dosage form of aqueous eye drops, there may be a problem with storage stability. Bromfenac is known to be unstable in an aqueous dosage form and has reportedly formed a red precipitate.

Thus, when preparing an ophthalmic composition containing moxifloxacin and bromfenac, it is essential to secure a formulation for improving the stability of moxifloxacin and bromfenac.

### [Related Art Reference]

### [Patent Documents]

(Patent Document 1) U.S. Registered Patent Publication No. 4910225
(Patent Document 2) International Unexamined Patent Publication No. 2001/10465

### Detailed Description of the Invention

### Technical Problem

The present invention may provide an ophthalmic composition which contains moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and β-cyclodextrin, derivatives thereof, or mixtures thereof.

The present invention may provide an antibacterial or anti-inflammatory method for an eye region, which includes administering to a subject (individual) an antibacterial or anti-inflammatory ophthalmic composition containing moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and β-cyclodextrin, derivatives thereof, or mixtures thereof.

The present invention may provide a method for preparing an ophthalmic composition with improved stability of moxifloxacin; bromfenac; or moxifloxacin and bromfenac, which includes mixing moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and β-cyclodextrin, derivatives thereof, or mixtures thereof.

The present invention may provide a method for stabilizing moxifloxacin; bromfenac; or moxifloxacin and bromfenac, which includes mixing moxifloxacin, or pharmaceutically acceptable salts thereof, or hydrates or solvates thereof; bromfenac, or pharmaceutically acceptable salts thereof, or hydrates or solvates thereof; polyoxyl hydrogenated castor oil; and β-cyclodextrin, derivatives thereof, or mixtures thereof.

The present invention may provide an ophthalmic composition which contains moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and hydroxypropyl-β-cyclodextrin.

The present invention may provide an antibacterial or anti-inflammatory method for an eye region, which includes administering to a subject an antibacterial or anti-inflammatory ophthalmic composition containing moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and hydroxypropyl-β-cyclodextrin.

The present invention may provide a method for preparing an ophthalmic composition with improved stability of moxifloxacin; bromfenac; or moxifloxacin and bromfenac, which includes mixing moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and hydroxypropyl-β-cyclodextrin.

The present invention may provide a method for stabilizing moxifloxacin; bromfenac; or moxifloxacin and bromfenac, which includes mixing moxifloxacin, or pharmaceutically acceptable salts thereof, or hydrates or solvates thereof; bromfenac, or pharmaceutically acceptable salts thereof, or hydrates or solvates thereof; polyoxyl hydrogenated castor oil; and hydroxypropyl-β-cyclodextrin.

### Technical Solution

The present invention provides an ophthalmic composition which contains moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and β-cyclodextrin, derivatives thereof, or mixtures thereof.

The present invention provides an ophthalmic composition which contains moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and hydroxypropyl-β-cyclodextrin.

The ophthalmic composition according to the present invention is prepared by appropriately mixing the above components, and has advantage of having more stable appearance than other commercially available eye drops. Thus, the ophthalmic composition may not produce precipitation during storage, and may not generate insoluble precipitation especially when stored for a long period of time. The composition may be stably maintained for a long period of time without precipitation even under stress condition as well as under room temperature condition, and thus may have remarkably excellent storage stability and may be economical without a need for additional conditions for improved storage stability. The ophthalmic composition according to the present invention may maintain a content of active ingredients during a storage period and may produce a low amount of related substances, thereby providing an advantage of excellent stability, and may be also physically and chemically stable, such as pH and osmolality. In addition, the ophthalmic composition according to the present invention may have an excellent sensation of instillation, and thus may cause little or no foreign body sensation, stinging and the like during instillation, and the composition may be also harmless to the human body, and thus may be advantageously used as a composition for ophthalmic formulation.

In the present invention, the above "moxifloxacin" is a type of fluoroquinolone-based antibacterial agent and a broad-spectrum antibiotic which is effective against both gram-positive bacteria and gram-negative bacteria, and inhibit cell replication by blocking enzymes such as topoisomerase required for isolating bacterial DNA.

The moxifloxacin may be contained in a therapeutically effective amount to achieve the purpose of antibacterial use. In the composition of the present invention, a content of moxifloxacin, pharmaceutically acceptable salts thereof, or hydrates or solvates thereof may be 0.1 w/v% or more, 0.2 w/v% or more, 0.3 w/v% or more, 0.4 w/v% or more, and 0.5 w/v% or more, and may be 1 w/v% or less, 0.9 w/v% or less, 0.8 w/v% or less, 0.7 w/v% or less, and 0.6 w/v% or less as moxifloxacin based on the total content of the ophthalmic composition. In the composition of the present invention, a content of moxifloxacin, pharmaceutically acceptable salts thereof, or hydrates or solvates thereof may be 0.1 to 1 w/v%, specifically 0.2 to 0.8 w/v%, more specifically 0.3 to 0.7 w/v%, and much more specifically 0.4 to 0.6 w/v%, and may be 0.5 w/v% as moxifloxacin based on the total content of the ophthalmic composition.

The ophthalmic composition of the present invention may show an antibacterial effect due to its nature of containing moxifloxacin, and thus may be advantageously used in preventing or treating eye diseases associated with infection with gram-negative and/or gram-positive bacteria or any other symptoms associated therewith.

In the present invention, "bromfenac" is a type of acetic acid-based nonsteroidal anti-inflammatory drug (NSAID), and the bromfenac inhibits cyclooxygenase activity, so as to exhibit analgesic and anti-inflammatory effects.

The bromfenac may be contained in a therapeutically effective amount to achieve the purpose of analgesic and/or anti-inflammatory use. In the composition of the present invention, a content of bromfenac, pharmaceutically acceptable salts thereof, or hydrates or solvates thereof may be 0.03 w/v% or more, 0.04 w/v% or more, 0.05 w/v% or more, and 0.06 w/v% or more, and may be 0.5 w/v% or less, 0.4 w/v% or less, 0.3 w/v% or less, 0.2 w/v% or less, and 0.1 w/v% or less as bromfenac based on the total content of the ophthalmic composition. In the composition of the present invention, a content of bromfenac, pharmaceutically acceptable salts thereof, or hydrates or solvates thereof may be 0.03 to 0.5 w/v%, specifically 0.04 to 0.4 w/v%, more specifically 0.05 to 0.3 w/v%, and much more specifically 0.06 to 0.2 w/v%, may be 0.06 to 0.1 w/v%, may be 0.06 w/v% as bromfenac based on the total content of the ophthalmic composition.

The ophthalmic composition of the present invention may show an analgesic and/or anti-inflammatory effect due to its nature of containing bromfenac, and thus may be advantageously used in preventing or treating inflammatory diseases of the external eye and the anterior eye, such as blepharitis, conjunctivitis, scleritis including episcleritis, inflammation after surgery, or the like, or any other symptoms associated therewith.

In the preparation of an ophthalmic formulation, bromfenac is unstable in an aqueous dosage form, and in the case of formulations containing polysorbate 80 as a nonionic surfactant, there may be a problem in that a red precipitate is generated after three weeks of storage, and it is not easy to prepare a stable ophthalmic composition containing bromfenac and moxifloxacin. Meanwhile, the composition according to the present invention may not produce insoluble precipitation under room temperature and stress conditions, and may be stored for a long period of time without an addition of special storage conditions.

Accordingly, the present invention may provide an ophthalmic composition which contains polyoxyl hydrogenated castor oil and β-cyclodextrin, derivatives thereof, or mixtures thereof with excellent stability of appearance.

In the present invention, the "polyoxyl hydrogenated castor oil" is also called polyoxyl hydrogenated castor oil as one type of solubilizer.

In the present invention, examples of the polyoxyl hydrogenated castor oil may include polyoxyl 40 hydrogenated castor oil, polyoxyl 60 hydrogenated castor oil, or polyoxyl 200 hydrogenated castor oil, but are not limited thereto.

In the present invention, the above polyoxyl 40 hydrogenated castor oil may be used interchangeably with HCO-40 or Cremophor RH40 in an equal sense.

A content of the polyoxyl hydrogenated castor oil may be 0.05 w/v% or more, 0.07 w/v% or more, and 0.1 w/v% or more, and may be 1.5 w/v% or less, 1 w/v% or less, 0.5 w/v% or less, 0.4 w/v% or less, and 0.3 w/v% or less of the total ophthalmic composition. A content of the polyoxyl hydrogenated castor oil may be 0.05 to 1.5 w/v%, 0.05 to 1.0 w/v%, specifically 0.05 to 0.4 w/v%, and more specifically 0.05 to 0.3 w/v% of the total ophthalmic composition. For example, a content of the polyoxyl 40 hydrogenated castor oil may be 0.05 w/v% or more, 0.07 w/v% or more, and 0.1 w/v% or more, and may be 1.5 w/v% or less, 1 w/v% or less, 0.5 w/v% or less, 0.4 w/v% or less, and 0.3 w/v% or less of the total ophthalmic composition. A content of the polyoxyl 40 hydrogenated castor oil may be 0.05 to 1.5 w/v%, 0.05 to 1.0 w/v%, specifically 0.05 to 0.4 w/v%, and more specifically 0.05 to 0.3 w/v%, and may be 0.1 w/v% of the total ophthalmic composition of the present invention.

In the present invention, the "β-cyclodextrin" is a cyclic oligosaccharide in which seven glucose molecules form an α-1,4-glycosidic bond.

In the present invention, a "derivative" of the β-cyclodextrin may be hydroxypropyl-β-cyclodextrin (HP-β-CD), dimethyl-β-cyclodextrin (DM-β-CD), 2-hydroxyethyl-β-cyclodextrin (2-HE-β-CD), trimethyl-β-cyclodextrin (TM-β-CD), and sulfobutylether-β-cyclodextrin (SBE-β-CD), in which any β-cyclodextrin-based derivative may not be limited thereto, and may specifically be hydroxypropyl-β-cyclodextrin.

A content of the β-cyclodextrin, derivatives thereof, or mixtures thereof may be 0.05 w/v% or more, 0.1 w/v% or more, 0.2 w/v% or more, 0.4 w/v% or more, and 0.5 w/v% or more, and may be 10 w/v% or less, 5 w/v% or less, 1 w/v% or less, 0.9 w/v% or less, 0.8 w/v% or less, 0.7 w/v% or less, and 0.6 w/v% or less of the total ophthalmic composition. A content of the β-cyclodextrin, derivatives thereof, or mixtures thereof may be 0.05 to 10 w/v%, 0.05 to 5 w/v%, specifically 0.1 to 0.9w/v%, more specifically 0.1 to 0.8 w/v%, and much more specifically 0.4 to 0.7 w/v%, and may be 0.5 w/v% of the total ophthalmic composition.

In the present invention, the "hydroxypropyl-β-cyclodextrin" may be a β-cyclodextrin derivative in which a hydroxypropyl group is introduced into β-cyclodextrin, and a content of the hydroxypropyl-β-cyclodextrin may be 0.05 w/v% or more, 0.1 w/v% or more, 0.2 w/v% or more, 0.4 w/v% or more, and 0.5 w/v% or more, and may be 10 w/v% or less, 5 w/v% or less, 1 w/v% or less, 0.9 w/v% or less, 0.8 w/v% or less, 0.7 w/v% or less, and 0.6 w/v% or less of the total ophthalmic composition. A content of the hydroxypropyl-β-cyclodextrin may be 0.05 to 10 w/v%, 0.05 to 5 w/v%, specifically 0.1 to 0.9 w/v%, more specifically 0.1 to 0.8 w/v%, and much more specifically 0.4 to 0.7 w/v%, and may be 0.5 w/v% of the total ophthalmic composition.

In one experimental example of the present invention, as a result of preparing an ophthalmic composition containing polyoxyl 40 hydrogenated castor oil and hydroxypropyl-β-cyclodextrin, which is a composition according to the present invention, and observing the stability of appearance after storing the composition for four weeks under room temperature and stress conditions, it has been confirmed that no precipitation occurs under room temperature and stress conditions all.

From the above experimental results, it could be seen that the ophthalmic composition containing both polyoxyl hydrogenated castor oil and hydroxypropyl-β-cyclodextrin has the stability of appearance under room temperature and stress conditions (Tables 2, 4 and 6, Experimental Example 1-3).

In the present invention, the ophthalmic composition may further contain a stabilizer. The stabilizer may include polyhydric alcohols including glycerol, sorbitol, mannitol, propylene glycol, etc.; cellulosic compounds including carboxymethylcellulose (CMC), hydroxypropylmethylcellulose (HPMC), hydroxyethylcellulose (HEC), etc.; polyvinyl-based compounds including polyvinyl alcohol (PVA), polyvinylpyrrolidone (PVP), etc.; polysaccharides including hyaluronic acid (HA), sodium hyaluronate, dextran, etc.; any combination thereof; or the like. In addition, edetic acid or sodium edetate, sodium edetate hydrate, tromethamine and the like may be used. Specifically, the stabilizer may be mannitol, sodium edetate hydrate, or a combination thereof. A content of the stabilizer may be 0.01 to 5 w/v% and 0.01 to 1 w/v% with regard to the content of the total composition.

In the present invention, the above ophthalmic composition may further contain a pH adjuster, isotonic agent, preservative, buffering agent, solvent or the like, but is not limited thereto.

The ophthalmic composition of the present invention has a pH suitable to be administered into eyes and the pH may be adjusted by a method known to those skilled in the art in order to obtain an appropriate pH.

The pH of the ophthalmic composition of the present invention may be 7 or more, specifically 7 to 10, 7 to 9, and more specifically 7.3 to 9.3.

The pH adjuster used herein may be sodium hydroxide, hydrochloric acid, etc., and may be added in an amount required for obtaining an appropriate pH according to a method known to those skilled in the art.

The above isotonic agent used herein may include: polyhydric alcohols including glycerol, sorbitol, mannitol, propylene glycol, etc.; salts including sodium chloride, potassium chloride, etc., but is not limited thereto, and a content thereof may be 0.01 to 10.0 w/v%, specifically 0.1 to 1.0 w/v% with regard to the content of the total composition.

The preservative of the present invention may include: quaternary ammonium compounds including benzalkonium chloride, benzethonium chloride, cetalkonium chloride, polyquaternium-1 (e.g., Polyquad^{®}), etc.; guanidine-based compounds including PHMB, chlorohexidine, etc.; chlorobutanol; mercury-based preservatives including thimerosal, phenylmercuric acetate, phenylmercuric nitrate and the like; and antioxidants including a stabilized oxychloro complex (e.g., Purite^{®}), p-hydroxybenzoate alkyls (e.g., methyl p-hydroxybenzoate (PM), etc.

The above preservative may be used by considering a side effect of eye drops, and a content of the preservatives may be 0.001 to 0.5 w/v%, preferably 0.001 to 0.05 w/v% with regard to the content of the total composition.

A buffering agent may be used herein without limitation, as long as the buffer is allowed to be used in eye drops as well. The buffering agent used herein may include an acetate buffer, citrate buffer, phosphate buffer (e.g., sodium hydrogen phosphate or hydrates thereof, and sodium dihydrogen phosphate or hydrates thereof), a boric acid buffer such as boric acid or salts thereof, borax, etc., but is not limited thereto. An amount of the above buffering agent used may be appropriately selected by those skilled in the art, and may be 0.001 to 10 w/v%, specifically 0.01 to 5.0 w/v%, and more specifically 0.1 to 2.0 w/v% with regard to the content of the total composition.

The solvent used herein may include sterile purified water, but is not limited thereto.

The present invention provides an antibacterial or anti-inflammatory method for an eye region, which includes administering to a subject an antibacterial or anti-inflammatory ophthalmic composition containing moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and β-cyclodextrin, derivatives thereof, or mixtures thereof.

The present invention provides an antibacterial or anti-inflammatory method for an eye region, which includes administering to a subject an antibacterial or anti-inflammatory ophthalmic composition containing moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and hydroxypropyl-β-cyclodextrin.

In the antibacterial or anti-inflammatory method for an eye region of the present invention, each component and the content thereof contained in the composition may be substantially the same as described above.

In the present invention, the "subject (individual)" may mean all the animals including humans, whose eye region is infected with bacteria or has a possibility of inflammation. The animals may include mammals such as a cow, horse, sheep, pig, goat, camel, antelope, dog, cat, etc., as well as humans, who need a treatment for other symptoms similar thereto, but are not limited thereto.

In the present invention, the "administration" means introducing the ophthalmic composition of the present invention into a subject by any appropriate method, and an administration route of the present invention may refer to being locally administered into eyeballs, considering that the composition corresponds to eye drops.

For example, the composition of the present invention may include moxifloxacin, pharmaceutically acceptable salts thereof, or hydrates or solvates thereof; bromfenac, pharmaceutically acceptable salts thereof, or hydrates or solvates thereof; polyoxyl 40 hydrogenated castor oil; hydroxypropyl-β-cyclodextrin; and water, but is not limited thereto. Each component and the content thereof contained in the composition may be substantially the same as described above.

For example, the composition of the present invention may include moxifloxacin, pharmaceutically acceptable salts thereof, or hydrates or solvates thereof; bromfenac, pharmaceutically acceptable salts thereof, or hydrates or solvates thereof; polyoxyl 40 hydrogenated castor oil; hydroxypropyl-β-cyclodextrin; sodium edetate or hydrates thereof; and water, but is not limited thereto. Each component and the content thereof contained in the composition may be substantially the same as described above.

The present invention provides a use of an ophthalmic composition for an antibacterial or anti-inflammatory of an eye region, which contains moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and β-cyclodextrin, derivatives thereof, or mixtures thereof.

The present invention provides a use of an ophthalmic composition for antibacterial or anti-inflammatory of an eye region, which contains moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and hydroxypropyl-β-cyclodextrin.

The present invention provides a use of an ophthalmic composition for the manufacture of a medicament for an antibacterial or anti-inflammatory of an eye region, which contains moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and β-cyclodextrin, derivatives thereof, or mixtures thereof.

The present invention provides a use of an ophthalmic composition for the manufacture of a medicament for an antibacterial or anti-inflammatory of an eye region, which contains moxifloxacin, pharmaceutically acceptable salts thereof, or hydrates or solvates thereof; bromfenac, pharmaceutically acceptable salts thereof, or hydrates or solvates thereof; polyoxyl hydrogenated castor oil; and hydroxypropyl-β-cyclodextrin. The present invention may provide a method for preparing an ophthalmic composition with improved stability of moxifloxacin; bromfenac; or moxifloxacin and bromfenac, which includes mixing moxifloxacin, or pharmaceutically acceptable salts thereof, or hydrates or solvates thereof; bromfenac, or pharmaceutically acceptable salts thereof, or hydrates or solvates thereof; polyoxyl hydrogenated castor oil; and β-cyclodextrin, derivatives thereof, or mixtures thereof.

The present invention may provide a method for preparing an ophthalmic composition with improved stability of moxifloxacin; bromfenac; or moxifloxacin and bromfenac, which includes mixing moxifloxacin, or pharmaceutically acceptable salts thereof, or hydrates or solvates thereof; bromfenac, or pharmaceutically acceptable salts thereof, or hydrates or solvates thereof; polyoxyl hydrogenated castor oil; and hydroxypropyl-β-cyclodextrin.

In the above preparation method of the present invention, each component contained in the composition and the content thereof may be the same as described above with respect to the ophthalmic composition, if not contradictory to each other.

The present invention may provide a method for stabilizing moxifloxacin; bromfenac; or moxifloxacin and bromfenac, which includes mixing moxifloxacin, or pharmaceutically acceptable salts thereof, or hydrates or solvates thereof; bromfenac, or pharmaceutically acceptable salts thereof, or hydrates or solvates thereof; polyoxyl hydrogenated castor oil; and β-cyclodextrin, derivatives thereof, or mixtures thereof.

The present invention may provide a method for stabilizing moxifloxacin; bromfenac; or moxifloxacin and bromfenac, which includes mixing moxifloxacin, or pharmaceutically acceptable salts thereof, or hydrates or solvates thereof; bromfenac, or pharmaceutically acceptable salts thereof, or hydrates or solvates thereof; polyoxyl hydrogenated castor oil; and hydroxypropyl-β-cyclodextrin.

In the above stabilization method of the present invention, each component contained in the composition and the content thereof may be the same as described above with respect to the ophthalmic composition, if not contradictory to each other.

The above method for preparing the ophthalmic composition may further include adding a pharmaceutically acceptable additive or carrier. The above pharmaceutically acceptable additive or carrier may be added without any other limitations in the process of preparing the ophthalmic composition.

The above method for stabilizing moxifloxacin; bromfenac; or moxifloxacin and bromfenac may further include adding a pharmaceutically acceptable additive or carrier. The above pharmaceutically acceptable additive or carrier may be added without any other limitations in the stabilization method.

The present invention may provide a method for preparing an ophthalmic composition with improved stability of moxifloxacin; bromfenac; or moxifloxacin and bromfenac, which includes mixing polyoxyl hydrogenated castor oil; β-cyclodextrin, derivatives or mixtures thereof; and pharmaceutically acceptable additives, and then adding moxifloxacin, or pharmaceutically acceptable salts thereof, or hydrates or solvates thereof; and bromfenac, or pharmaceutically acceptable salts thereof, or hydrates or solvates thereof.

The present invention may provide a method for stabilizing moxifloxacin; bromfenac; or moxifloxacin and bromfenac, which includes mixing polyoxyl hydrogenated castor oil; β-cyclodextrin, derivatives or mixtures thereof; and pharmaceutically acceptable additives, and then adding moxifloxacin, or pharmaceutically acceptable salts thereof, or hydrates or solvates thereof; and bromfenac, or pharmaceutically acceptable salts thereof, or hydrates or solvates thereof.

In the preparation method and stabilization method, the pH of the composition may be adjusted after mixing one or more active ingredients.

Regarding the ophthalmic composition according to the present invention, the use of the compositions, the preparation method, and the antibacterial or anti-inflammatory method including administering the compositions, the above compositions, the use thereof, the preparation method thereof or descriptions applied to the method may be equally applied to the respective compositions, uses or methods, if not contradictory to each other.

### Advantageous Effects

The ophthalmic composition according to the present invention may be useful as eye drops which show excellent appearance stability, secure long-term storage stability, and are easy to store while exhibiting excellent antibacterial and anti-inflammatory effects. The ophthalmic composition according to the present invention maintains a content of active ingredients well and has excellent stability with a small amount of related substances. In addition, the ophthalmic composition is physicochemically stable in terms of pH, osmolality and the like, and has an excellent sensation of instillation.

### Mode for Invention

Hereinafter, the configuration and effects of the present invention will be described in more detail through Examples. However, the following Examples are provided only for the purpose of illustrating the present invention, and thus the content of the present invention is not limited thereto.

### Experimental Example 1: Experiment on stability of appearance of composition of Example 1 and compositions of Comparative Examples 1 to 3

Components and contents of the composition of Example 1 according to the present invention and the compositions of Comparative Examples 1 to 3 are shown in table 1 below.

**[Table 1]**

| (Unit: w/v%) | | | | |
|---|---|---|---|---|
| Component | Example 1 | Comparative Example 1 | Comparative Example 2 | Comparative Example 3 |
| Moxifloxacin hydrochloride | 0.545 | 0.545 | 0.545 | 0.545 |
| Bromfenac sodium hydrate | 0.069 | 0.069 | 0.069 | 0.069 |
| Boric acid (boric acid, H₃BO₃) | 1.1 | 1.1 | 1.1 | 1.1 |
| Borax (Sodium tetraborate decahydrate) | 1.1 | 1.1 | 1.1 | 1.1 |
| HP-β-CD | 0.5 | - | 0.5 | - |
| Polyoxyl 40 hydrogenated castor oil | 0.1 | - | - | 0.1 |
| Sodium edetate hydrate | - | - | - | q. s. (quantum sufficit) |
| pH | About 8.3 | About 8.3 | About 8.3 | About 8.3 |
| Sterile purified water | QS 100 w/v% | QS 100 w/v% | QS 100 w/v% | QS 100 w/v% |

The pH of the compositions of Example 1 and Comparative Examples 1 to 3 was adjusted by adding NaOH, respectively.

The compositions of above Example according to the present invention and Comparative Examples were prepared according to the following method so as to accomplish the compositions as shown in table 1.

A composition was prepared in such a way that moxifloxacin hydrochloride and bromfenac sodium hydrate (1.5 hydrates) were added and homogeneously dissolved into a solution which was obtained by mixing boric acid, borax, HP-β-CD, and polyoxyl 40 hydrogenated castor oil (and sodium edetate hydrate in the case of Comparative Example 3) in accordance with the contents shown in table 1.

The stability of appearance of the composition of Example 1 and compositions of Comparative Examples 1 to 3 was confirmed by observing whether insoluble precipitation occurred or not in the composition of above Example 1 and the compositions of Comparative Examples 1 to 3 under room temperature (25°C, relative humidity 40%) and stress conditions (Example 1, Comparative Examples 1 and 2: 70°C, relative humidity 55%, and Comparative Example 3: 55°C, relative humidity 75%) over a storage period (four weeks), and the results are summarized as follows.

**[Table 2]**

| | Example 1 | Comparative Example 1 | Comparative Example 2 | Comparative Example 3 |
|---|---|---|---|---|
| Room temperature condition (occurrence of precipitation) | × | ○ | × | ○ |
| Stress condition (occurrence of precipitation) | × | ○ | ○ | ○ |

The composition of Example 1 showed the remarkably excellent stability of appearance without precipitation even after four weeks under room temperature and stress conditions all.

Meanwhile, fine precipitation occurred under room temperature condition, and red precipitation was generated under stress condition in Comparative Example 1. In addition, precipitation did not occur under room temperature condition, but the color turned dark brown and slightly reddish precipitation was generated under stress condition in Comparative Example 2. Further, yellow crystalline precipitation occurred under room temperature condition, and yellow insoluble crystalline precipitation was generated even under stress condition in Comparative Example 3.

Thus, the compositions of Comparative Examples 1 to 3 did not have the improved stability of appearance due to occurrence of precipitation either under room temperature or stress condition, whereas in the composition of Example 1 according to the present invention, any insoluble precipitation was not produced under room temperature and stress conditions all, thus indicating that the stability of appearance is improved.

### Experimental Example 2: Experiment on stability of appearance of composition of Example 2

Components and contents of the composition of Example 2 according to the present invention are shown in table 3 below.

**[Table 3]**

| (Unit: w/v%) | |
|---|---|
| Component | Example 2 |
| Moxifloxacin hydrochloride | 0.545 |
| Bromfenac sodium hydrate | 0.066 |
| Boric acid (boric acid, H₃BO₃) | 1.1 |
| Borax (Sodium tetraborate decahydrate) | 1.1 |
| HP-β-CD | 0.5 |
| Polyoxyl 40 hydrogenated castor oil | 0.1 |
| pH | About 8.3 |
| Sterile purified water | QS 100 w/v% |

The pH of the composition of Example 2 was adjusted by adding NaOH.

The composition of above Example 2 according to the present invention was prepared according to the following method so as to accomplish the composition as shown in table 3.

A composition was prepared in such a way that moxifloxacin hydrochloride and bromfenac sodium hydrate (1.5 hydrates) were added and homogeneously dissolved into a solution which was obtained by mixing boric acid, borax, HP-β-CD, and polyoxyl 40 hydrogenated castor oil in accordance with the contents shown in table 3.

The stability of appearance of the composition of Example 2 was confirmed by observing whether insoluble precipitation occurred or not in the composition of above Example 2 under room temperature condition (25°C, relative humidity 40%) and stress condition (70°C, relative humidity 55%) over a storage period (four weeks), and the results are summarized as follows.

**[Table 4]**

| | Example 2 |
|---|---|
| Room temperature condition (occurrence of precipitation) | X |
| Stress condition (occurrence of precipitation) | X |

The composition of Example 2 showed the improved stability of appearance without any insoluble precipitation even after four weeks under room temperature and stress conditions all.

### Experimental Example 3: Experiment on stability of appearance of compositions of Examples 3 to 6

Components and contents of the compositions of Examples 3 to 6 according to the present invention are shown in table 5 below.

**[Table 5]**

| (Unit: w/v%) | | | | |
|---|---|---|---|---|
| Component | Example 3 | Example 4 | Example 5 | Example 6 |
| Moxifloxacin hydrochloride | 0.545 | 0.545 | 0.545 | 0.545 |
| Bromfenac sodium hydrate | 0.066 | 0.066 | 0.066 | 0.066 |
| Boric acid (boric acid, H₃BO₃) | 1.1 | 1.1 | 1.1 | 1.1 |
| Borax (Sodium tetraborate decahydrate) | 1.1 | 1.1 | 1.1 | 1.1 |
| HP-β-CD | 0.05 | 5.0 | 0.05 | 5.0 |
| Polyoxyl 40 hydrogenated castor oil | 0.05 | 0.05 | 1.0 | 1.0 |
| pH | 8.35 | 8.34 | 8.37 | 8.33 |

The pH of the composition of each Example was adjusted by adding NaOH.

The compositions of above Examples 3 to 6 according to the present invention were prepared according to the following method so as to accomplish the compositions as shown in table 5.

A composition was prepared in such a way that moxifloxacin hydrochloride and bromfenac sodium hydrate (1.5 hydrates) were added and homogeneously dissolved into a solution which was obtained by mixing boric acid, borax, HP-β-CD, and polyoxyl 40 hydrogenated castor oil in accordance with the contents shown in table 5.

The stability of appearance of the compositions of Examples 3 to 6 was confirmed by observing whether insoluble precipitation occurred or not under stress condition (55°C) over a storage period (three weeks), and the results are summarized as follows.

**[Table 6]**

| | Example 3 | Example 4 | Example 5 | Example 6 |
|---|---|---|---|---|
| Occurrence of precipitation | X | X | X | X |

As a result of the experiment, it could be confirmed that the compositions of Examples 3 to 6 had the excellent stability of appearance without precipitation.

### Experimental Example 4: Experiment on stability of composition of Example 7

The composition of Example 7 was prepared in such a way that moxifloxacin hydrochloride and bromfenac sodium hydrate (1.5 hydrates) were added and homogeneously dissolved into a solution which was obtained by mixing boric acid, borax, HP-β-CD, polyoxyl 40 hydrogenated castor oil and sodium edetate hydrate in accordance with the contents shown in table 7, after which pH was adjusted with NaOH.

**[Table 7]**

| (Unit: w/v%) | |
|---|---|
| Component | Example 7 |
| Moxifloxacin hydrochloride | 0.545 |
| Bromfenac sodium hydrate | 0.066 |
| Boric acid | 1.1 |
| Borax | 1.1 |
| HP-β-CD | 0.5 |
| Polyoxyl 40 hydrogenated castor oil | 0.1 |
| Sodium edetate hydrate | 0.02 |
| pH | 8.3 |

The composition of Example 7 prepared as above was stored under stress condition (55°C, relative humidity 75%) for four weeks, after which the appearance of the composition was observed and the pH, osmolality, content of active ingredients (moxifloxacin and bromfenac sodium hydrate), and amount of related substances generated with respect to the composition were measured.

It was confirmed that the composition of Example 7 stably maintained appearance without occurrence of precipitation or color change under stress condition, and the pH and osmolality thereof were also stable without great change (Table 8). In addition, it was confirmed that the composition of Example 7 had very excellent stability, as the content of active ingredients was also stably maintained and the amount of related substances generated was also less (Table 8).

**[Table 8]**

| | Week 0 | Week 4 |
|---|---|---|
| Appearance | Transparent yellow | Transparent yellow |
| pH | 8.30 | 8.30 |
| Osmolality (mOsmol/kg) | 285 | 293 |
| Content of moxifloxacin (%) | 100 | 96.31 |
| Content of bromfenac sodium hydrate (%) | 100 | 99.32 |
| Total amount of related substances generated with respect to moxifloxacin (%) | N.D. | 0.03 |
| Total amount of related substances generated with respect to bromfenac sodium hydrate (%) | N.D. | 0.54 |

### Experimental Example 5: Experiment on stability of compositions of Examples 8 to 11

The compositions of Examples 8 to 11 were prepared in such a way that moxifloxacin hydrochloride and bromfenac sodium hydrate (1.5 hydrates) were added and homogeneously dissolved into a solution which was obtained by mixing boric acid; borax; HP-β-CD, SBE-β-CD or β-CD; polyoxyl 40 hydrogenated castor oil; and sodium edetate hydrate in accordance with the components and the contents shown in table 9, after which pH was adjusted with NaOH.

**[Table 9]**

| (Unit: w/v%) | | | | |
|---|---|---|---|---|
| Component | Example 8 | Example 9 | Example 10 | Example 11 |
| Moxifloxacin hydrochloride | 0.545 | 1.09 | 1.09 | 1.09 |
| Bromfenac sodium hydrate | 0.066 | 0.55 | 0.55 | 0.033 |
| Boric acid | 1.1 | 1.1 | 1.1 | 1.1 |
| Borax | 1.1 | 1.1 | 1.1 | 1.1 |
| HP-β-CD | 10.0 | 10.0 | - | - |
| sBE-β-CD | - | - | 10.0 | - |
| β-CD | - | - | - | 0.5 |
| Polyoxyl 40 hydrogenated castor oil | 0.05 | 1.5 | 0.1 | 0.1 |
| Sodium edetate hydrate | 0.02 | 0.02 | 0.02 | 0.02 |
| pH | 8.29 | 8.33 | 8.31 | 8.28 |

The compositions of Examples 8 to 11 prepared as above were stored under stress condition (55°C, relative humidity 75%) for two weeks, after which the appearance of the compositions were observed and the pH, osmolality, content of active ingredients (moxifloxacin and bromfenac sodium hydrate), and amount of related substances generated with respect to the composition were measured.

It was confirmed that the compositions of Examples 8 to 11 stably maintained appearance under stress condition, and the pH and osmolality thereof were also stable without major change (Table 10). In addition, it was confirmed that the compositions of Examples 8 to 11 were very stable compositions, as the content of active ingredients was also stably maintained and the amount of related substances generated was also less (Table 10).

**[Table 10]**

| | Composition | Week 0 | Week 2 |
|---|---|---|---|
| Appearance | Example 8 | Transparent yellow | Transparent yellow |
| | Example 9 | Transparent yellow | Transparent dark yellow |
| | Example 10 | Transparent yellow | Transparent dark yellow |
| | Example 11 | Transparent yellow | Transparent dark yellow |
| pH | Example 8 | 8.29 | 8.35 |
| | Example 9 | 8.33 | 8.25 |
| | Example 10 | 8.31 | 8.28 |
| | Example 11 | 8.28 | 8.32 |
| Osmolality (mOsmol/kg) | Example 8 | 400 | 399 |
| | Example 9 | 466 | 450 |
| | Example 10 | 611 | 586 |
| | Example 11 | 302 | 299 |
| Content of moxifloxacin (%) | Example 8 | 100 | 104.54 |
| | Example 9 | 100 | 95.65 |
| | Example 10 | 100 | 97.62 |
| | Example 11 | 100 | 100.05 |
| Content of bromfenac sodium hydrate (%) | Example 8 | 100 | 99.47 |
| | Example 9 | 100 | 104.88 |
| | Example 10 | 100 | 104.37 |
| | Example 11 | 100 | 103.57 |
| Total amount of related substances generated with respect to moxifloxacin (%) | Example 8 | N.D. | N.D. |
| | Example 9 | 0.02 | 0.08 |
| | Example 10 | 0.02 | 0.12 |
| | Example 11 | 0.02 | 0.10 |
| Total amount of related substances generated with respect to bromfenac sodium hydrate (%) | Example 8 | N.D. | 0.20 |
| | Example 9 | N.D. | 0.21 |
| | Example 10 | N.D. | 0.24 |
| | Example 11 | N.D. | 0.25 |

While specific portions of the present invention have been described in detail above, it is apparent to those skilled in the art that such detailed descriptions are set forth to illustrate exemplary embodiments only, but are not construed to limit the scope of the present invention.

Thus, it should be understood that the substantial scope of the present invention is defined by the accompanying claims and equivalents thereto.

## Claims

1. An ophthalmic composition comprising:
(1) moxifloxacin, pharmaceutically acceptable salts thereof, or hydrates or solvates thereof;
(2) bromfenac, pharmaceutically acceptable salts thereof, or hydrates or solvates thereof;
(3) polyoxyl hydrogenated castor oil; and
(4) β-cyclodextrin, derivatives thereof, or mixtures thereof.

2. The ophthalmic composition of claim 1, wherein the polyoxyl hydrogenated castor oil is any one selected from the group consisting of polyoxyl 40 hydrogenated castor oil, polyoxyl 60 hydrogenated castor oil, and polyoxyl 200 hydrogenated castor oil.

3. The ophthalmic composition of claim 2, wherein the polyoxyl hydrogenated castor oil is polyoxyl 40 hydrogenated castor oil.

4. The ophthalmic composition of claim 1, wherein a derivative of the β-cyclodextrin is any one selected from the group consisting of hydroxypropyl-β-cyclodextrin (HP-β-CD), dimethyl-β-cyclodextrin (DM-β-CD), 2-hydroxyethyl-β-cyclodextrin (2-HE-β-CD), trimethyl-β-cyclodextrin (TM-β-CD), and sulfobutylether-β-cyclodextrin (SBE-β-CD).

5. The ophthalmic composition of claim 4, wherein the derivative of the β-cyclodextrin is hydroxypropyl-β-cyclodextrin.

6. The ophthalmic composition of claim 1, wherein a content of the polyoxyl hydrogenated castor oil is 0.05 to 1.5 w/v% of the total composition.

7. The ophthalmic composition of claim 1, wherein a content of the β-cyclodextrin, derivatives thereof, or mixtures thereof is 0.05 to 10 w/v% of the total composition.

8. The ophthalmic composition of claim 1, wherein the composition further comprises at least one component selected from the group consisting of a buffering agent, stabilizer, pH adjuster and solvent.

9. The ophthalmic composition of claim 1, wherein a pH of the composition is 7 to 10.

10. The ophthalmic composition of claim 1, wherein the composition is an antibacterial or anti-inflammatory composition.

11. The ophthalmic composition of claim 1, wherein a content of the moxifloxacin, pharmaceutically acceptable salts thereof, or hydrates or solvates thereof is 0.1 to 1 w/v% of the total composition as moxifloxacin.

12. The ophthalmic composition of claim 1, wherein a content of the bromfenac, pharmaceutically acceptable salts thereof, or hydrates or solvates thereof is 0.03 to 0.5 w/v% of the total composition as bromfenac.

13. An antibacterial or anti-inflammatory method for an eye region, the method comprising: administering the ophthalmic composition of any one of claims 1 to 9, 11 and 12 into a subject.

14. A use of the ophthalmic composition of any one of claims 1 to 9, 11 and 12 for the manufacture of a medicament for an antibacterial or anti-inflammatory of an eye region.

15. A use of the ophthalmic composition of any one of claims 1 to 9, 11 and 12 for antibacterial or anti-inflammatory of an eye region.
